# EUROPEAN PATENT APPLICATION

(11) **EP 4 686 721 A1**
(43) Date of publication of application: **04.02.2026**
(21) Application number: 24192456.2
(22) Date of filing: 01.08.2024
(51) Int. Cl.: C07D 489/08

(54) **CRYSTALLINE NALOXONE-DIMETHYLFORMAMIDE SOLVATE, METHODS FOR ITS PREPARATION AND ITS USE AS A REAGENT IN A METHOD OF MAKING NALOXONE**

(71) Applicant: Siegfried AG, 4800 Zofingen (CH)
(72) Inventor: Petzold, Daniel, 6260 Hintermoos (CH)
(74) Representative: Eisenführ Speiser

(57) **Abstract**

Described herein is a crystalline naloxone-dimethylformamide solvate and methods of making a first and a second amount thereof, as well as methods of making naloxone or a naloxone salt, involving said crystalline naloxone-dimethylformamide solvate. Further described herein is the use of dimethylformamide for preparing said crystalline naloxone-dimethylformamide solvate and the use of said crystalline naloxone-dimethylformamide solvate as a reagent for i.a. purifying naloxone. Moreover, it is described a crystalline naloxone and a mixture comprising dimethylformamide and said crystalline naloxone-dimethylformamide solvate.
Naloxone has the following formula I:

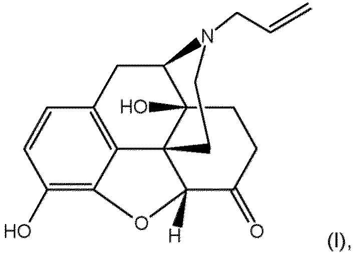

## Description

The present invention relates to a crystalline naloxone-dimethylformamide solvate and methods of making a first and a second amount thereof, as well as to methods of making naloxone or a naloxone salt, involving said crystalline naloxone-dimethylformamide solvate. The present invention also relates to the use of dimethylformamide for preparing said crystalline naloxone-dimethylformamide solvate and to the use of said crystalline naloxone-dimethylformamide solvate as a reagent for i.a. purifying naloxone. Moreover, the present invention relates to a crystalline naloxone and to a mixture comprising dimethylformamide and said crystalline naloxone-dimethylformamide solvate.

Naloxone of formula I 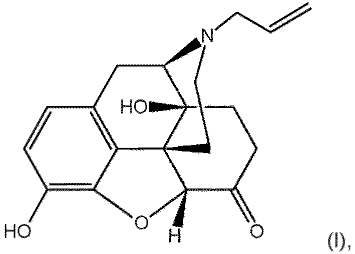 is a known opioid antagonist, often used as a medication to reverse or reduce the effects of opioids, in particular for restoring breathing after an opioid overdose. Effects of naloxone as an opioid antagonist begin within minutes when given intravenously, injected into a muscle or as a nasal spray. It has been reported that naloxone can block the effects of opioids for up to 90 minutes. Naloxone is commonly used in the form of its dihydrated hydrochloride salt, which is usually prepared from the free base (see formula I above), but other salts of naloxone are also known.

One known synthesis of naloxone comprises a step where noroxymorphone of formula II 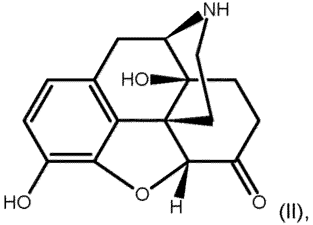 is reacted with an alkylating reagent, e.g. allyl bromide, in the presence of a base. A respective synthesis is e.g. described in document WO 2015/171353 A2. For isolating naloxone from a respective synthesis in larger quantities which would be suitable for practical or industrial, in particular pharmaceutical, purposes, complex separation and purification steps are currently necessary. Such complex separation and purification steps lead to high energy consumption, long synthesis times and the undesired production of a relatively high amount of waste materials. In addition, the final yield of naloxone from a respective synthesis is not yet completely satisfactory.

In the light of the existing prior art, there is therefore a need for a simple, straightforward, efficient and sustainable method of making naloxone, preferably as an isolated compound in larger quantities, which is ideally suited for pharmaceutical use in terms of purity, and for reagents facilitating such desirable method of making naloxone.

Correspondingly, it was a primary object of the present invention to provide a simple, straightforward, efficient and sustainable method of making naloxone, including providing a reagent for facilitating such method, which method would make available naloxone as isolated compound in larger quantities and ideally in a grade of purity suitable for pharmaceutical use. Further objects of the present invention concern providing methods of making and using said reagent.

It has now been found that the primary object and other objects of the present invention can be accomplished by a crystalline naloxone-dimethylformamide solvate.

The present inventor has recognized that said crystalline naloxone-dimethylformamide solvate, as is further described herein, is a valuable reagent for facilitating a simple, straightforward, efficient and sustainable method of making naloxone (as is also described herein in more detail), which method would make available naloxone as isolated compound in larger quantities and ideally in a grade of purity which makes it suited for pharmaceutical use.

The invention as well as preferred variants and preferred combinations of parameters, properties and elements thereof are defined in the appended claims. Preferred aspects, details, modifications and advantages of the present invention are also defined and explained in the following description and in the figures and examples shown below.

Preferred is a crystalline naloxone-dimethylformamide solvate according to the present invention as described herein (or a crystalline naloxone-dimethylformamide solvate according to the present invention as described herein as being preferred), which, in an X-ray powder diffraction diagram at 25 °C and using Cu Ka-radiation, displays at least three of the following reflections, quoted as 20-values in °: 6.55 ± 0.2, 10.90 ± 0.2, 13.00 ± 0.2, 14.06 ± 0.2, 15.7 ± 0.2, 19.10 ± 0.2, 21.09 ± 0.2, 21.77 ± 0.2, 22.79 ± 0.2, and 26.11 ± 0.2;
preferably wherein the crystalline naloxone-dimethylformamide solvate displays all of the following reflections, quoted as 20-values in °: 6.55 ± 0.2, 10.90 ± 0.2, 13.00 ± 0.2, 14.06 ± 0.2, 15.7 ± 0.2, 19.10 ± 0.2, 21.09 ± 0.2, 21.77 ± 0.2, 22.79 ± 0.2, and 26.11 ± 0.2.

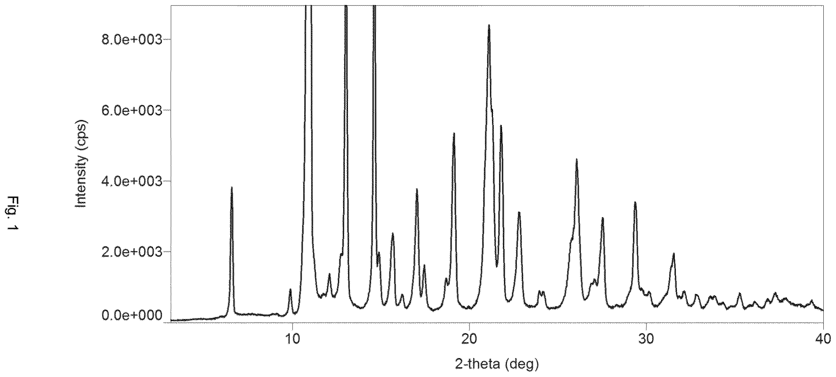

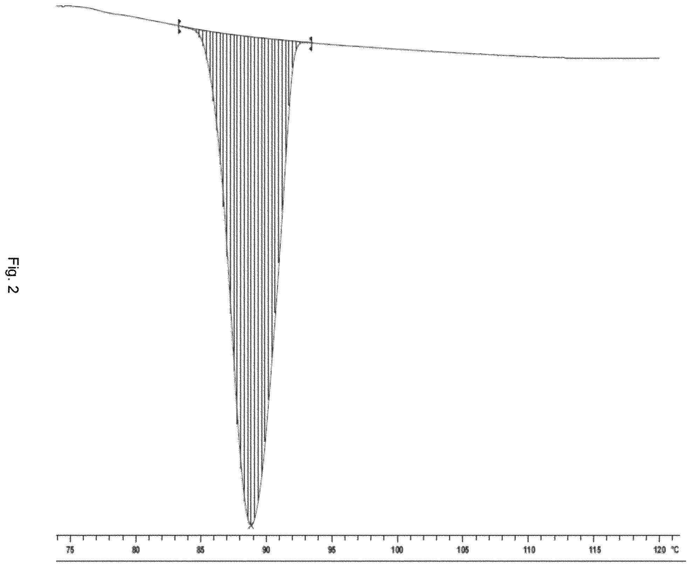

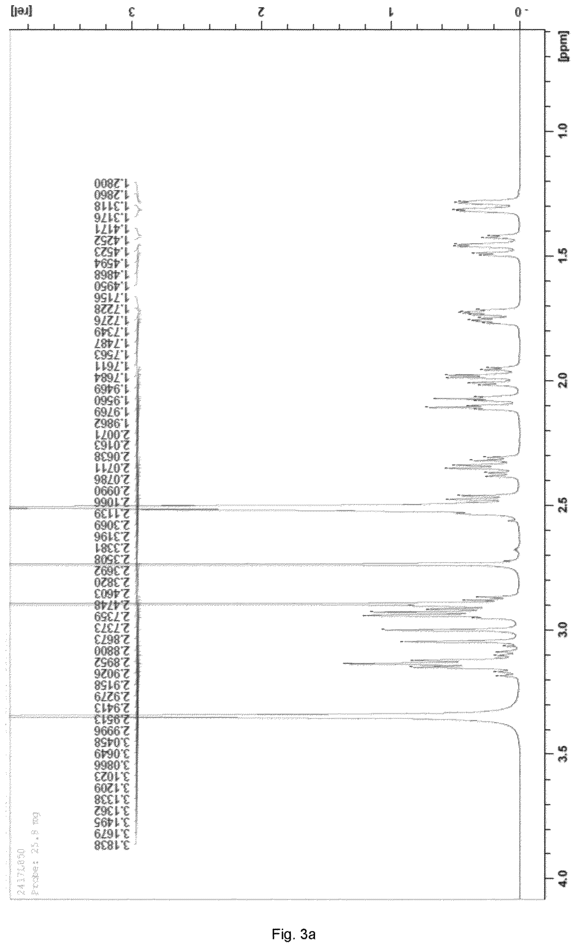

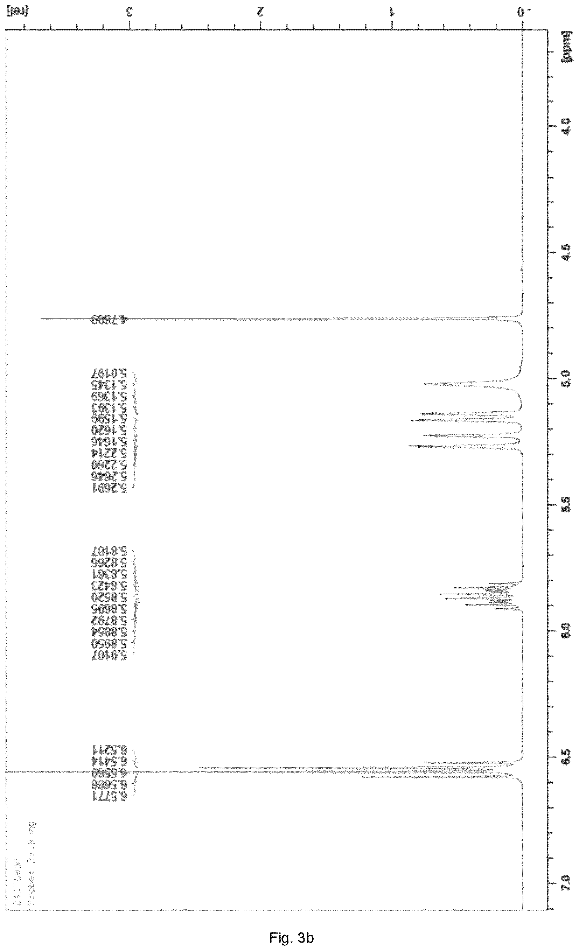

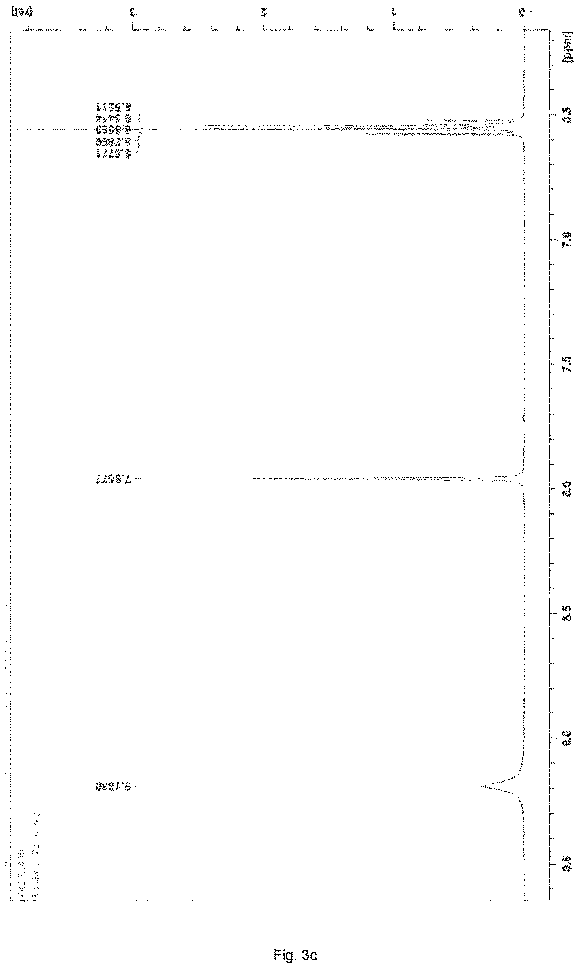

Preferred is also a crystalline naloxone-dimethylformamide solvate according to the present invention as described herein (or a crystalline naloxone-dimethylformamide solvate according to the present invention as described herein as being preferred), characterized or further characterized by:
- an X-ray powder diffraction diagram which is essentially similar to the X-ray powder diffraction diagram shown in Fig. 1,
   preferably wherein the crystalline naloxone-dimethylformamide solvate is characterized by an X-ray powder diffraction diagram as shown in Fig. 1;
   and/or
- a differential scanning calorimetry diagram as shown in Fig. 2;
   and/or
- a melting point in the range of from ≥ 84 °C to ≤ 95 °C, preferably of from ≥ 87 °C to ≤ 92 °C, determined by differential scanning calorimetry at a heating rate of 4 °C/min;
   and/or
- an ¹H-NMR diagram as shown in Figs. 3a, 3b and 3c, all referring to an ¹H-NMR spectrum measured in DMSO-d₆ at 23 °C;
   and/or
- an ¹H-NMR spectrum measured in DMSO-d₆ at 23 °C, which shows at least the following signals in ppm: 9.2; 8.0; 6.5; 5.9; 5.24; 5.15; 5.0; 4.8; 3.1; 3.0; 3.0-2.8; 2.7; 2.5-2.4; 2.3; 2.1; 2.0; 1.7; 1.5; 1.3.

Preferably, the ¹H-NMR spectrum measured in DMSO-d₆ at 23 °C of the crystalline naloxone-dimethylformamide solvate according to the present invention as described herein shows at least the following signals in ppm, as are further characterized here below:
9.2 (brs, 1H); 8.0 (s, 1H); 6.5 (q, 2H); 5.9 (m, 1H); 5.24 (dd, 1H); 5.15 (dd, 1H); 5.0 (brs, 1H); 4.8 (s, 1H); 3.1 (m, 2H); 3.0 (d, 1H); 3.0-2.8 (m, 2H + s, 3H); 2.7 (d, 3H); 2.5-2.4 (partially below DMSO signal, m, 1H); 2.3 (dt, 1H); 2.1 (dt, 1H); 2.0 (dt, 1H); 1.7 (dq, 1H); 1.5 (dt, 1H); 1.3 (dt, 1H),
where "brs" means "broad singlet", "s" means "singlet", "q" means "quartet", "m" means "multiplet", "d" means "doublet", "dd" means "double doublet", "dt" means "double triplet" and "dq" means "double quartet", as is common in the technical field.

Furthermore is preferred a crystalline naloxone-dimethylformamide solvate according to the present invention as described herein (or a crystalline naloxone-dimethylformamide solvate according to the present invention as described herein as being preferred) comprising or consisting of:
- a total amount of naloxone in the range of from ≥ 50 to ≤ 90 wt.-%, preferably of from ≥ 75 to ≤ 85 wt.-%, based on the total weight of the crystalline naloxone-dimethylformamide solvate,
   and/or (preferably "and")
- a total amount of dimethylformamide in the range of from ≥ 10 to ≤ 50 wt.-%, preferably of from ≥ 15 to ≤ 25 wt.-%, based on the total weight of the crystalline naloxone-dimethylformamide solvate.

The present invention also pertains to a method of making naloxone of formula I (see above), comprising the steps:
S1) providing or preparing noroxymorphone of formula II (see above), or a salt or a solvate thereof,
S2) reacting the noroxymorphone of formula II, or the salt or solvate thereof, in a liquid reaction medium comprising dimethylformamide, in the presence of a base, preferably an organic amine base, with an allyl halide, to receive a first reaction mixture;
S3) to receive a second amount of crystalline naloxone-dimethylformamide solvate as a precipitated solid,
i) seeding the first reaction mixture received in step S2) with a first amount of a crystalline naloxone-dimethylformamide solvate according to the present invention as described herein (or a crystalline naloxone-dimethylformamide solvate according to the present invention as described herein as being preferred)
   or
ii) stirring the first reaction mixture received in step S2) for a time period sufficient for a second amount of crystalline naloxone-dimethylformamide solvate (according to the present invention as described herein or as described herein as being preferred) to precipitate as a solid,
   and/or
   adjusting the temperature of the first reaction mixture received in step S2) to a temperature in the range of from -60 °C to 30 °C, preferably of from -15 °C to 15 °C and more preferably of from -15 °C to 5 °C,
and

| | |
|---|---|
| S4) | adding to the crystalline naloxone-dimethylformamide solvate received in step S3) as precipitated solid a solvent or solvent mixture suitable to release naloxone of formula I from said crystalline naloxone-dimethylformamide solvate. |

All aspects of the present invention discussed herein in the context of the crystalline naloxone-dimethylformamide solvate according to the present invention as defined herein apply *mutatis mutandis* to the method of making naloxone of formula I according to the present invention, as defined herein, and *vice versa.*

It was found in own experiments that the method of making naloxone according to the present invention as defined above and further described herein, is simple, straightforward, efficient and sustainable. In particular, the method according to the present invention, when compared with similar methods from the prior art, allows to reduce the number of steps and correspondingly the overall time required for producing, isolating and purifying naloxone; allows to reduce the overall consumption of energy required for the production of naloxone, in particular for naloxone of high purity (in particular of a grade of purity which is suited for pharmaceutical use); allows to reduce the amount of waste material presently associated with the production of naloxone and also allows to increase the yield of naloxone.

In step S1) of the method of making naloxone of formula I according to the present invention as described herein, noroxymorphone, or a salt or a solvate thereof, is provided or prepared. Noroxymorphone is a known opioid, which is both a metabolite of oxymor-phone and oxycodone and is often manufactured specifically as an intermediate in the production of narcotic antagonists such as naltrexone. Methods for the synthesis of noroxymorphone are known and are e.g. described in EP 0 158 476 A1; in US 5,668,285; in WO 2022/144911, or by F. Sommer et al. in ACS Sustainable Chem. Eng. 10, 27 (2022) 8988-8996.

In step S2) of the method of making naloxone of formula I according to the present invention as described herein, noroxymorphone of formula II (see above), or the salt or solvate thereof, is reacted in a liquid reaction medium comprising dimethylformamide, in the presence of a base, with an allyl halide. Preferably, the reaction in step S2) is carried out at a temperature in the range of from -60 °C to 120 °C, more preferably of from 40 °C to 65 °C and even more preferably of from 45 °C to 60 °C, e.g. at 55 °C. The liquid reaction medium preferably comprises a mixture of dimethylformamide with one or more solvents which are inert under the reaction conditions (i.e. the base and the allyl halide used in step S2) are preferably not counted as part of the "liquid reaction medium"), where, however, the concentration of dimethylformamide in the liquid reaction medium is high enough to allow precipitation of the crystalline naloxone-dimethylformamide solvate in later reaction steps. Preferably, the liquid reaction medium comprises a total amount of ≥ 80 wt.-%, more preferably of ≥ 85 wt.-% and even more preferably of ≥ 90 wt.-% of dimethylformamide, relative to the total weight of the liquid reaction medium. In one particularly preferred variant of the method of the present invention, (solely) dimethylformamide is used as the liquid reaction medium. Suitable bases for use in step S2) are common organic amine bases, preferably sterically hindered organic amine bases like diisopropylethylamine.

In variant "i)" of step S3) of the method of making naloxone of formula I according to the present invention as defined herein, the first reaction mixture received in step S2) is seeded with a first amount of a crystalline naloxone-dimethylformamide solvate. Preferably, the first amount of crystalline naloxone-dimethylformamide solvate is (considerably) smaller than the second amount of crystalline naloxone-dimethylformamide solvate received in step S3). Preferably, the first reaction mixture is seeded with a total first amount of crystalline naloxone-dimethylformamide solvate in the range of from ≥ 0.01 to ≤ 2.5 wt.-%, preferably of from ≥ 0.025 to ≤ 1.0 wt.-%, more preferably of from ≥ 0.04 to ≤ 0.5 wt.-%, relative to the total amount of noroxymorphone provided or prepared in step S1) of the present method.

Variant "i)" of step S3) as defined above is preferred: It was found in own experiments that in said variant "i)" most of the (crude) naloxone formed in step S2) and present in the first reaction mixture precipitates as crystalline naloxone-dimethylformamide solvate and naloxone can later be obtained in high purity and high yield from said crystalline naloxone-dimethylformamide solvate so prepared.

In variant "ii)" of step S3) as defined above, crystalline naloxone-dimethylformamide solvate may precipitate spontaneously (without being seeded with a first amount of said crystalline naloxone-dimethylformamide solvate as in variant "i)" defined above), when the first reaction mixture is stirred for a time period sufficient to facilitate such precipitation and/or when the first reaction mixture is cooled to a temperature in the range of from -60 °C to 30 °C, preferably of from -15 °C to 15 °C and more preferably of from -15 °C to 5 °C. However, variant "ii)" of step S3) as defined above does usually not yield or provide crystalline naloxone-dimethylformamide solvate in a similar high quantity and/or purity as variant "i)" of step S3) of the method according to the present invention. The time period which is sufficient to facilitate precipitation of crystalline naloxone-dimethylformamide solvate from the first reaction mixture depends from different parameters, comprising temperature of the first reaction mixture.

In step S4) of the method of making naloxone of formula I according to the present invention as described herein, a solvent or solvent mixture is added to the crystalline naloxone-dimethylformamide solvate received in step S3) as precipitated solid, wherein said solvent or solvent mixture is suitable to release naloxone of formula I from said crystalline naloxone-dimethylformamide solvate (for suitable solvents or solvent mixtures see below). It was an unexpected finding by the present inventors that, by adding said suitable solvent or solvent mixture to the crystalline naloxone-dimethylformamide solvate received in step S3) as precipitated solid, naloxone can - preferably quantitatively - be released from the precipitated crystalline naloxone-dimethylformamide solvate, while the naloxone so released can be isolated in higher purity and in higher yield than in a comparable process, where naloxone was not first precipitated as crystalline naloxone-dimethylformamide solvate and subsequently released again from said crystalline naloxone-dimethylformamide solvate by the method described herein.

Preferred is a method of making naloxone according to the present invention as described herein (or a method of making naloxone according to the present invention as described herein as being preferred), wherein
- step S3) comprises variant i) and preferably further comprises adjusting the temperature (preferably cooling) of the seeded first reaction mixture received in step S2) to a temperature in the range of from -60 °C to 120 °C, preferably of from -20 °C to 40 °C, more preferably cooling the seeded first reaction mixture to a temperature in the range of from -15 °C to 20 °C, even more preferably of from -15 °C to 15 °C and yet even more preferably of from -15 °C to 5 °C;
   and/or
- the allyl halide in step S2) is selected from the group consisting of allyl chloride, allyl bromide, allyl iodide and mixtures thereof, preferably wherein the allyl halide in step S2) comprises or is allyl bromide;
   and/or
- the first reaction mixture in step S2) and/or the seeded first reaction mixture in step S3) comprises 3O-allylnaloxone of formula III 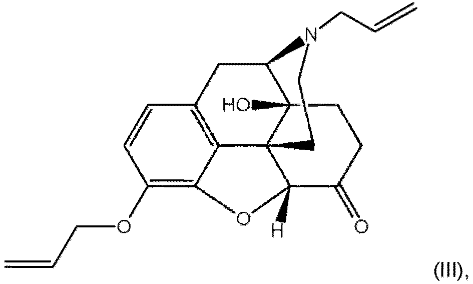 preferably as an impurity.

It has been found in own experiments that variant "i)" of step S3) delivers even better results in terms of yield and purity of naloxone finally obtained from the overall method of making naloxone according to the present invention, when the temperature of the seeded first reaction mixture received in step S2) is adjusted to a temperature in the range or in the preferred ranges as defined here above.

In the method of making naloxone according to the present invention as described herein (or in a method of making naloxone according to the present invention as described herein as being preferred), naloxone is prepared in step S2) from noroxymorphone, where noroxymorphone is reacted with an allyl halide. One typical side product of this reaction is in many cases 3O-allylnaloxone of formula III. It has been found in own experiments that the method of making naloxone according to the present invention is particularly suited for providing naloxone in high purity, more in particular for providing naloxone with a particularly small proportion of 3O-allylnaloxone as an impurity. Ideally, naloxone prepared by the method of making naloxone according to the present invention is free of 3O-allylnaloxone. The method of making naloxone according to the present invention as described herein, i.e. involving a step where naloxone is first precipitated as crystalline naloxone-dimethylformamide solvate and subsequently released again from said crystalline naloxone-dimethylformamide solvate, is therefore particularly suited for reducing the amount of 3O-allylnaloxone in the naloxone obtained as a product.

Furthermore is preferred a method of making naloxone according to the present invention as described herein (or a method of making naloxone according to the present invention as described herein as being preferred), wherein
- the solvent or solvent mixture suitable to release naloxone of formula I from the precipitated solid crystalline naloxone-dimethylformamide solvate in step S4) is selected from the group consisting of
   - water,
   - an alkyl alcohol having 1 to 4 carbon atoms, preferably selected from the group consisting of methanol, ethanol, 1-propanol, isopropanol, n-butanol, 2-methyl-1-propanol and mixtures thereof, more preferably the alkyl alcohol having 1 to 4 carbon atoms comprises or is 1-propanol;
      and
   - mixtures thereof
   and/or
- step S4) comprises adjusting the pH of the mixture received after addition of the solvent or solvent mixture suitable to release naloxone of formula I from said crystalline naloxone-dimethylformamide solvate, to a value in the range of from 8.5 to 10, preferably of from 9.0 to 9.5, preferably measured at 0 °C;
   and/or
- the method further comprises the step
   S5) separating solid naloxone of formula I as received from step S4) and optionally drying it.

Preferably, the solvent or solvent mixture suitable to release naloxone from the precipitated solid crystalline naloxone-dimethylformamide solvate in step S4) is selected from the group as defined above. Preferred for said purpose is a solvent mixture comprising water and 1-propanol or water. Most preferred is using water as (sole) solvent for releasing naloxone from the precipitated solid crystalline naloxone-dimethylformamide solvate in step S4).

A variant of the method of making of naloxone according to the present invention is preferred, wherein in step S4), after the addition of the solvent or solvent mixture suitable to release naloxone of formula I from said crystalline naloxone-dimethylformamide solvate, the pH of the resulting mixture is adjusted to a value in the range of from 8.5 to 10, preferably of from 9.0 to 9.5, preferably measured at 0 °C and wherein preferably the resulting mixture with the pH so adjusted is held at a temperature in the range of from -5 °C to 15 °C, preferably of from -5 °C to 5 °C.

It was found in own experiments that, under the reaction and work-up conditions as described herein, or under the preferred reaction and work-up conditions, as described herein, the naloxone released from the solid crystalline naloxone-dimethylformamide solvate can be separated from the reaction mixture by filtration in high yield and purity. Without wishing to be bound by theory, it is assumed that the solvent or solvent mixture suitable to release naloxone from the precipitated solid crystalline naloxone-dimethylformamide solvate in step S4) washes out or generally removes the dimethylformamide from said crystalline naloxone-dimethylformamide solvate to yield naloxone as a (preferably crystalline) solid, which can simply be filtered off and e.g. washed with suitable solvents or liquids for further purification, and optionally subsequently dried.

It was further found in own experiments that the naloxone received as a crystalline solid from the method of making of naloxone according to the present invention shows a particular X-ray powder diffraction diagram, which X-ray powder diffraction diagram is different from a respective X-ray powder diffraction diagram of a naloxone which is received from a different method of making, preferably wherein naloxone is re-crystallized from ethyl acetate.

The present invention therefore also concerns a crystalline naloxone, which
- in an X-ray powder diffraction diagram at 25 °C and using Cu Ka-radiation, displays at least three of the following reflections, quoted as 20-values in °: 10.46 ± 0.2, 11.23 ± 0.2, 11.68 ± 0.2, 13.61 ± 0.2, 14.53 ± 0.2, 16.27 ± 0.2, 16.63 ± 0.2, 17.45 ± 0.2, 18.05 ± 0.2, 20.31 ± 0.2, 20.95 ± 0.2,
   preferably wherein the crystalline naloxone displays all of the following reflections, quoted as 20-values in °: 10.46 ± 0.2, 11.23 ± 0.2, 11.68 ± 0.2, 13.61 ± 0.2, 14.53 ± 0.2, 16.27 ± 0.2, 16.63 ± 0.2, 17.45 ± 0.2, 18.05 ± 0.2, 20.31 ± 0.2, 20.95 ± 0.2,
   and/or
- is obtainable or obtained by a method of making naloxone according to the present invention as described herein (or by a method of making naloxone according to the present invention as described herein as being preferred).

All aspects of the present invention discussed herein in the context of the crystalline naloxone-dimethylformamide solvate according to the present invention as defined herein and/or in the context of the method of making naloxone of formula I according to the present invention as defined herein apply *mutatis mutandis* to the crystalline naloxone according to the present invention as defined here above, and *vice versa.*

The present invention furthermore pertains to a method of making a naloxone salt, preferably naloxone hydrochloride, comprising the steps

| | |
|---|---|
| S6) | providing crystalline naloxone according to the present invention as defined herein, or preparing naloxone of formula I by the method of making naloxone according to the present invention as described herein (or a method of making naloxone according to the present invention as described herein as being preferred), and |
| S7) | reacting the naloxone of formula I as received from step S6) with an acid, preferably with hydrochloric acid. |

All aspects of the present invention discussed herein in the context of the crystalline naloxone-dimethylformamide solvate according to the present invention as defined herein and/or in the context of the method of making naloxone of formula I according to the present invention as defined herein and/or in the context of the crystalline naloxone according to the present invention as defined herein apply *mutatis mutandis* to the method of making a naloxone salt according to the present invention as defined here above, and *vice versa.*

In step S7), the naloxone of formula I as received from step S6) is reacted with an acid, preferably with hydrochloric acid. Suitable acids are such acids which, together with the naloxone of formula I, form a physiologically acceptable naloxone salt. Another acid which may be used in step S7) for forming a (physiologically acceptable) naloxone salt is saccharin, which, together with naloxone of formula I, forms naloxone saccharinate.

Under a further aspect, the present invention also pertains to a method of making a first amount of a crystalline naloxone-dimethylformamide solvate, preferably a crystalline naloxone-dimethylformamide solvate according to the present invention as defined herein, comprising the steps

| | |
|---|---|
| S11) | providing or preparing naloxone, or a salt or a solvate thereof, wherein any provided or prepared solvate of naloxone is different from crystalline naloxone-dimethylformamide solvate, |
| S12) | dissolving or suspending the naloxone, or the salt or solvate thereof, provided or prepared in step S11) in a liquid reaction medium comprising dimethylformamide, for a time sufficient so that the crystalline naloxone-dimethylformamide is formed and |
| S13) | preferably isolating the crystalline naloxone-dimethylformamide which has formed in step S12) and optionally drying it. |

All aspects of the present invention discussed herein in the context of the crystalline naloxone-dimethylformamide solvate according to the present invention as defined herein and/or in the context of the method of making naloxone of formula I according to the present invention as defined herein and/or in the context of the crystalline naloxone according to the present invention as defined herein and/or in the context of the method of making a naloxone salt according to the present invention as defined herein apply *mutatis mutandis* to the method of making a first amount of a crystalline naloxone-dimethylformamide solvate according to the present invention as defined here above, and *vice versa.*

In step S12) of the method of making a first amount of a crystalline naloxone-dimethylformamide solvate according to the present invention as described herein, the naloxone, or the salt or solvate thereof is dissolved or suspended in a liquid reaction medium comprising dimethylformamide. The liquid reaction medium preferably comprises a mixture of dimethylformamide with one or more solvents which are inert under the reaction conditions, where, however, the concentration of dimethylformamide in the liquid reaction medium is high enough to allow precipitation of the crystalline naloxone-dimethylformamide. Preferably, the liquid reaction medium comprises a total amount of ≥ 80 wt.-%, more preferably of ≥ 85 wt.-% and even more preferably of ≥ 90 wt.-% of dimethylformamide, relative to the total weight of the liquid reaction medium. In one particularly preferred variant of the method of the present invention, (solely) dimethylformamide is used as the liquid reaction medium. Preferably, the temperature of the mixture received in step S12) after the naloxone, or the salt or solvate thereof, has been dissolved or suspended in the liquid reaction medium, is adjusted to a temperature in the range of from -60 °C to 120 °C, more preferably in the range of from 18 °C to 30°C. Preferably, the said mixture is also intimately mixed (preferably vortexed), preferably for a time period in the range of from 1 to 15 min., more preferably of from 2 to 10 min. Further preferably, the concentration of the naloxone, or the salt or solvate thereof, in the liquid reaction medium in step S12) is in the range of from ≥ 20 to ≤ 60 % wt./wt., more preferably of from ≥ 38 to ≤ 42 % wt./wt.

In step S13) of the method of making a first amount of a crystalline naloxone-dimethylformamide solvate according to the present invention as described here above, the crystalline naloxone-dimethylformamide which has formed in step S12) is preferably isolated by filtration. Optional drying of the isolated crystalline naloxone-dimethylformamide is preferably done at a temperature in the range of from 0 °C to 120 °C, more preferably of from 35 °C to 50 °C.

The present invention thus also pertains to a crystalline naloxone-dimethylformamide solvate, obtainable or obtained by a method of making a first amount of a crystalline naloxone-dimethylformamide solvate according to the present invention as defined herein (or according to a method of making a first amount of a crystalline naloxone-dimethylformamide solvate according to the present invention as described herein as being preferred).

All aspects of the present invention discussed herein in the context of the crystalline naloxone-dimethylformamide solvate according to the present invention as defined herein and/or in the context of the method of making naloxone of formula I according to the present invention as defined herein and/or in the context of the crystalline naloxone according to the present invention as defined herein and/or in the context of the method of making a naloxone salt according to the present invention as defined herein and/or in the context of the method of making a first amount of a crystalline naloxone-dimethylformamide solvate according to the present invention as defined herein apply *mutatis mutandis* to the crystalline naloxone-dimethylformamide solvate, obtainable or obtained by a method of making a first amount of a crystalline naloxone-dimethylformamide solvate according to the present invention as defined here above, and *vice versa.*

Moreover, the present invention pertains to a method of making a second or further amount of a crystalline naloxone-dimethylformamide solvate, preferably a crystalline naloxone-dimethylformamide solvate according to the present invention as defined above (or a crystalline naloxone-dimethylformamide solvate according to the present invention as defined above as being preferred), comprising the steps:
S21) providing or preparing, in a liquid reaction medium comprising dimethylformamide, naloxone of formula I, as defined above, or a salt or a solvate thereof, wherein any provided or prepared solvate of naloxone is different from (crystalline) naloxone-dimethylformamide solvate,
   to receive a second reaction mixture;
   and
S22) seeding the second reaction mixture received in step S21) with a first amount of crystalline naloxone-dimethylformamide solvate, preferably according to the present invention as defined above (or with a crystalline naloxone-dimethylformamide solvate according to the present invention as defined above as being preferred), and optionally cooling the seeded second reaction mixture, to receive a second amount of crystalline naloxone-dimethylformamide solvate as a precipitated solid.

All aspects of the present invention discussed herein in the context of the crystalline naloxone-dimethylformamide solvate according to the present invention as defined herein and/or in the context of the method of making naloxone of formula I according to the present invention as defined herein and/or in the context of the crystalline naloxone according to the present invention as defined herein and/or in the context of the method of making a naloxone salt according to the present invention as defined herein and/or in the context of the method of making a first amount of a crystalline naloxone-dimethylformamide solvate according to the present invention as defined herein and/or in the context of the crystalline naloxone-dimethylformamide solvate, obtainable or obtained by a method of making a first amount of a crystalline naloxone-dimethylformamide solvate according to the present invention as defined herein apply *mutatis mutandis* to the method of making a second or further amount of a crystalline naloxone-dimethylformamide solvate according to the present invention as defined here above, and *vice versa.*

In step S21) of the method of making a second amount of a crystalline naloxone-dimethylformamide solvate according to the present invention as described here above, naloxone (of formula I as described above) is provided or prepared in a liquid reaction medium comprising dimethylformamide. The liquid reaction medium preferably comprises a mixture of dimethylformamide with one or more solvents which are inert under the reaction conditions, where, however, the concentration of dimethylformamide in the liquid reaction medium is high enough to allow precipitation of the crystalline naloxone-dimethylformamide. Preferably, the liquid reaction medium comprises a total amount of ≥ 80 wt.-%, more preferably of ≥ 85 wt.-% and even more preferably of ≥ 90 wt.-% of dimethylformamide, relative to the total weight of the liquid reaction medium. In one particularly preferred variant of the method of the present invention, (solely) dimethylformamide is used as the liquid reaction medium.

Preferably, the second reaction mixture received in said step S21) is initially heated to a temperature in the range of from 35 °C to 120 °C, more preferably of from 35 °C to 65 °C, even more preferably in the range of from 40 °C to 60 °C, to facilitate or to advance dissolution of the naloxone of formula I, or the salt or solvate thereof, in the liquid reaction medium. Preferably, the temperature of the total weight of second reaction mixture is subsequently adjusted to a temperature in the range of from 10 °C to 40 °C, more preferably in the range of from 32 °C to 36 °C.

Further preferably, the concentration of the naloxone, or the salt or solvate thereof, in the liquid reaction medium in step S21) is in the range of from ≥ 25 to ≤ 45 % wt./wt., more preferably of from ≥ 31 to ≤ 35 % wt./wt.

In step S22) of the method of making a second amount of a crystalline naloxone-dimethylformamide solvate according to the present invention as described here above, the second reaction mixture received in step S21) is seeded with a first amount of crystalline naloxone-dimethylformamide solvate. Preferably, the second reaction mixture is seeded with a total first amount of crystalline naloxone-dimethylformamide solvate in the range of from ≥ 0.025 to ≤ 5 wt.-%, preferably of from ≥ 0.1 to ≤ 2.5 wt.-%, more preferably of from ≥ 0.25 to ≤ 1.5 wt.-%, relative to the total amount of naloxone of formula I provided or prepared in step S21) of the present method. In step S22), the seeded second reaction mixture is preferably cooled, preferably to a temperature in the range of from -60 °C to 15 °C, more preferably of from -20 °C to 15 °C, even more preferably in the range of from -15 °C to 5 °C, to facilitate or to advance the precipitation of the second or further amount of crystalline naloxone-dimethylformamide solvate. The second or further amount of crystalline naloxone-dimethylformamide solvate may then be isolated, preferably by filtration, optionally washed with suitable solvents or liquids and optionally dried.

As was explained above, 3O-allylnaloxone of formula III is a typical side product of a known reaction for preparing naloxone from noroxymorphone, i.e. by reacting noroxymorphone with an allyl halide. When naloxone is provided or prepared from such a reaction, the second reaction mixture in step S21) and/or the seeded second reaction mixture in step S22) of the present method usually comprises 3O-allylnaloxone as an impurity.

As was also stated above, the method of making naloxone according to the present invention, i.e. involving a step where naloxone is first precipitated as crystalline naloxone-dimethylformamide solvate and subsequently released again from said crystalline naloxone-dimethylformamide solvate by the method described herein, is particularly effective for purifying naloxone, in particular for reducing the amount of 3O-allylnaloxone in the naloxone finally obtained from the method of making naloxone according to the present invention.

Preferred is therefore a method of making a second amount of a crystalline naloxone-dimethylformamide solvate according to the present invention as described herein (or a method of making a second amount of a crystalline naloxone-dimethylformamide solvate according to the present invention as described herein as being preferred), wherein the second reaction mixture in step S21) and/or the seeded second reaction mixture in step S22) comprises 3O-allylnaloxone of formula III as defined above, preferably as an impurity.

Under a still further aspect, and in view of the explanations given here above, the present invention also pertains to the use of a crystalline naloxone-dimethylformamide solvate, preferably a crystalline naloxone-dimethylformamide solvate according to the present invention as defined here above (or of a crystalline naloxone-dimethylformamide solvate according to the present invention as defined here above as being preferred), as a reagent for crystallizing, recrystallizing and/or purifying naloxone or a salt or solvate thereof (where preferably the solvate of naloxone is different from crystalline naloxone-dimethylformamide solvate).

All aspects of the present invention discussed herein in the context of the crystalline naloxone-dimethylformamide solvate according to the present invention as defined herein and/or in the context of the method of making naloxone of formula I according to the present invention as defined herein and/or in the context of the method of making a naloxone salt according to the present invention as defined herein and/or in the context of the crystalline naloxone according to the present invention as defined herein and/or in the context of the method of making a first amount of a crystalline naloxone-dimethylformamide solvate according to the present invention as defined herein and/or in the context of the crystalline naloxone-dimethylformamide solvate, obtainable or obtained by a method of making a first amount of a crystalline naloxone-dimethylformamide solvate according to the present invention as defined herein and/or in the context of the method of making a second or further amount of a crystalline naloxone-dimethylformamide solvate according to the present invention as defined herein apply *mutatis mutandis* to the use of a crystalline naloxone-dimethylformamide solvate according to the present invention as defined here above, and *vice versa.*

It has been found in own experiments that the crystalline naloxone-dimethylformamide solvate according to the present invention as defined herein is excellently suited as a reagent, in particular as a seeding material, for facilitating, advancing and/or supporting crystallization of naloxone in the form of said crystalline naloxone-dimethylformamide solvate. Said crystalline naloxone-dimethylformamide solvate can subsequently very easily be converted again into the naloxone of formula I (as is further explained above in more detail), while said naloxone of formula I so received again is obtained in high yield and characterized by its high purity. Furthermore, purifying naloxone via crystallization of its corresponding dimethylformamide solvate according to the methods of the present invention as disclosed herein makes several time- and energy-consuming purifying steps for naloxone (which are presently usually applied in methods of making naloxone) unnecessary and superfluous.

Under an additional aspect, the present invention further concerns the use of dimethylformamide for preparing a crystalline naloxone-dimethylformamide solvate from naloxone, preferably the crystalline naloxone-dimethylformamide solvate according to the present invention as defined herein (or a crystalline naloxone-dimethylformamide solvate according to the present invention as defined herein as being preferred).

All aspects of the present invention discussed herein in the context of the crystalline naloxone-dimethylformamide solvate according to the present invention as defined herein and/or in the context of the method of making naloxone of formula I according to the present invention as defined herein and/or in the context of the method of making a naloxone salt according to the present invention as defined herein and/or in the context of the crystalline naloxone according to the present invention as defined herein and/or in the context of the method of making a first amount of a crystalline naloxone-dimethylformamide solvate according to the present invention as defined herein and/or in the context of the crystalline naloxone-dimethylformamide solvate, obtainable or obtained by a method of making a first amount of a crystalline naloxone-dimethylformamide solvate according to the present invention as defined herein and/or in the context of the method of making a second or further amount of a crystalline naloxone-dimethylformamide solvate according to the present invention as defined herein and/or in the context of the use of a crystalline naloxone-dimethylformamide solvate according to the present invention as defined herein apply *mutatis mutandis* to the use of dimethylformamide for preparing a crystalline naloxone-dimethylformamide solvate from naloxone according to the present invention as defined here above, and *vice versa.*

The present invention then also pertains to a mixture, preferably a suspension, comprising dimethylformamide and a crystalline naloxone-dimethylformamide solvate, preferably a crystalline naloxone-dimethylformamide solvate according to the present invention as defined herein (or according to the present invention as defined herein as being preferred).

All aspects of the present invention discussed herein in the context of the crystalline naloxone-dimethylformamide solvate according to the present invention as defined herein and/or in the context of the method of making naloxone of formula I according to the present invention as defined herein and/or in the context of the method of making a naloxone salt according to the present invention as defined herein and/or in the context of the crystalline naloxone according to the present invention as defined herein and/or in the context of the method of making a first amount of a crystalline naloxone-dimethylformamide solvate according to the present invention as defined herein and/or in the context of the crystalline naloxone-dimethylformamide solvate, obtainable or obtained by a method of making a first amount of a crystalline naloxone-dimethylformamide solvate according to the present invention as defined herein and/or in the context of the method of making a second or further amount of a crystalline naloxone-dimethylformamide solvate according to the present invention as defined herein and/or in the context of the use of a crystalline naloxone-dimethylformamide solvate according to the present invention as defined herein and/or in the context of the use of dimethylformamide for preparing a crystalline naloxone-dimethylformamide solvate from naloxone according to the present invention as defined herein apply *mutatis mutandis* to the mixture comprising dimethylformamide and a crystalline naloxone-dimethylformamide solvate according to the present invention as defined here above, and *vice versa.*

A respective mixture comprising dimethylformamide and a crystalline naloxone-dimethylformamide solvate according to the present invention can preferably be obtained by (i) the method of naloxone of formula I according to the present invention as disclosed herein, by (ii) the method of making a first amount of a crystalline naloxone-dimethylformamide solvate according to the present invention as disclosed herein and/or by (iii) the method of making a second or further amount of a crystalline naloxone-dimethylformamide solvate according to the present invention as disclosed herein.

### Figures:

The present invention is further described and illustrated with reference to the figures appended herewith.
- Fig. 1:: Fig. 1 shows an X-ray powder diffraction diagram of the crystalline naloxone-dimethylformamide solvate according to the present invention, measured at 25 °C and using Cu Kα-radiation.
- Fig. 2:: Fig. 2 shows a differential scanning calorimetry diagram of the crystalline naloxone-dimethylformamide solvate according to the present invention, measured at a heating rate of 4 °C/min. The y-axis shows heat flow in units of Wg⁻¹.
- Fig. 3a:: Fig. 3a shows the section between 0.6 and 4.0 ppm of an ¹H-NMR spectrum of the crystalline naloxone-dimethylformamide solvate according to the present invention, measured in DMSO-d₆ at 23 °C.
- Fig. 3b:: Fig. 3b shows the section between 4.0 and 7.0 ppm of an ¹H-NMR spectrum of the crystalline naloxone-dimethylformamide solvate according to the present invention, measured in DMSO-d₆ at 23 °C.
- Fig. 3c:: Fig. 3c shows the section between 6.5 and 9.5 ppm of an ¹H-NMR spectrum of the crystalline naloxone-dimethylformamide solvate according to the present invention, measured in DMSO-d₆ at 23 °C.

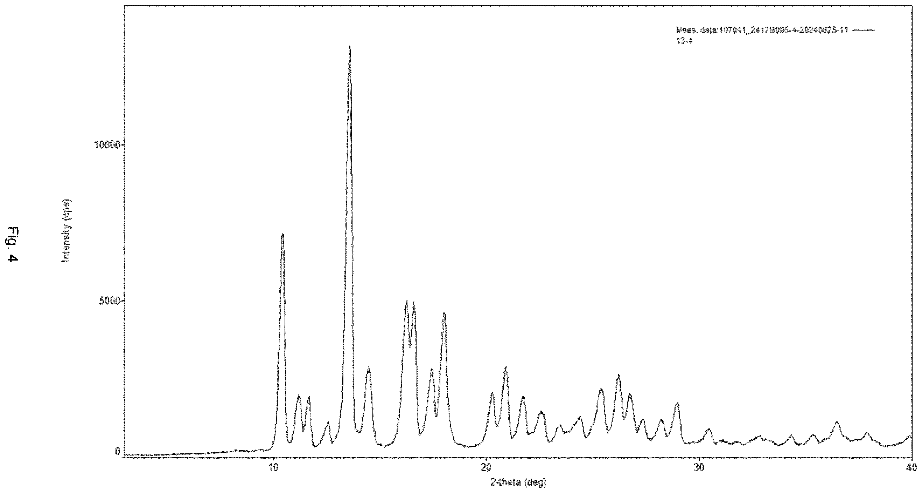

- Fig. 4:: Fig. 4 shows an X-ray powder diffraction diagram of the crystalline naloxone according to the present invention, measured at 25 °C and using Cu Kα-radiation.

### Examples:

The following examples are meant to further explain and illustrate the present invention without limiting its scope.

### Example 1: Method of making a first amount of crystalline naloxone-dimethylformamide solvate according to the present invention

1.0 g of crude naloxone and 1.5 g of dimethylformamide ("DMF") were placed in a test tube at about 23 °C (room temperature, RT). The resulting mixture (initially a suspension) was intimately mixed (vortexed) for 5 minutes. The solid substance obtained was filtered off, rinsed with several drops of cold DMF and sucked to dryness. The filter cake was then dried at 40 °C (jacket temperature) overnight.

The crystalline naloxone-dimethylformamide solvate was obtained as grayish solid in 41 % yield (relative to the amount of crude naloxone used as starting material) and > 99.5 area-% purity, as measured by high performance liquid chromatography ("HPLC", measured as sum of naloxone and DMF peaks).

### Example 2: Method of making a second amount of crystalline naloxone-dimethylformamide solvate according to the present invention

100 g of crude naloxone were suspended in 200 g DMF in an inerted reactor at about 23 °C (RT). The resulting suspension was heated to 50 °C until most or all of the solid was dissolved. The solution so obtained was cooled to about 35 °C, seeded with crystalline naloxone dimethylformamide solvate (0.5 g, for preparation see Example 1) and stirred at about 35 °C for one hour.

The suspension so obtained was cooled to -10 °C within four hours and was stirred for another twelve hours at this temperature. The suspension was then filtered, the filter cake was washed with DMF (50 g, previously cooled to -10 °C) and sucked to dryness. The remaining solid substance was dried overnight at 25 °C (jacket temperature) with a flow of nitrogen in a drying oven.

Crystalline naloxone dimethylformamide solvate was so obtained as a grayish to brownish solid in 70 % yield (relative to the amount of crude naloxone used as starting material) and 99.9 area-% HPLC purity (measured as sum of naloxone + DMF peaks).

### Example 3: Method of making naloxone according to the present invention

Noroxymorphone (100 g), butylated hydroxytoluene (2,6-di-tert-butyl-4-methylphenol, "BHT"; 1.53 g), diisopropylethylamine (45.9 g) and DMF (190 g) were placed in an inerted reactor and stirred at about 23 °C for 10 minutes. Allyl bromide (43.8 g) was added from a dosing vessel over 30 minutes to the reaction mixture so obtained. The dosing vessel was rinsed with DMF (15 g) and the rinsing liquid was combined with the reaction mixture. The resulting suspension was heated to 55 °C within one hour and stirred for an additional 1.5 hours.

The mixture thus obtained was transferred to a second reactor, the first reactor and the transfer lines were rinsed with DMF (30 g) and the rinsing liquids were combined with the mixture. The mixture including the rinsing liquids was then cooled to 32 °C, seeded with crystalline naloxone-dimethylformamide solvate (0.05 g, for preparation see Example 2) and stirred for about 60 minutes to result in a thin suspension. Said thin suspension was cooled to 0 °C over three hours and then purified water (135 g) was added to it at this temperature over two hours. When all the water was added, the pH of the resulting mixture was adjusted to 9.3 at 0 °C and the mixture was then stirred for another three hours at 0 °C.

The suspension so obtained was filtered and the filter cake was washed twice with water (2x 100 g, tempered to 25 °C) and (via the reactor) twice with a mixture of 1-propanol (2x 67 g, cooled to 0 °C) and water (2x 33 g cooled to 0 °C). Naloxone was obtained as a grayish to brownish solid and dried in a drying oven at 55 °C and a pressure of p ≤ 5000 Pa for 16 hours. After drying was completed, the yield of naloxone was 85 % (relative to the amount of noroxymorphone used as starting material) and its purity was 99.7 area-% (measured by HPLC).

### Example 4: Method of making naloxone not according to the present invention (for comparison)

Noroxymorphone (100 g), butylated hydroxytoluene (1.53 g), diisopropylethylamine (45.9 g) and DMF (190 g) were placed in an inerted reactor and stirred at about 23 °C for 10 minutes. Allyl bromide (43.8 g) was added from a dosing vessel over 30 minutes to the reaction mixture so obtained. The dosing vessel was rinsed with DMF (15 g) and the rinsing liquid was combined with the reaction mixture. The resulting suspension was heated to 55 °C within one hour and stirred for an additional 1.5 hours.

To the mixture thus obtained, purified water (100 g) was added at 55 °C over 60 minutes. Subsequently, the resulting mixture was seeded with crystalline naloxone (0.5 g) and stirred for about 60 minutes to result in a suspension. Said suspension was cooled to 0 °C over three hours and then purified water (100 g) was added to it at this temperature over 30 minutes. When all the water was added, the pH of the resulting mixture was adjusted to 9.2 at 0 °C and the mixture was then stirred for another 16 hours at 0 °C.

The suspension so obtained was filtered and the filter cake was washed twice with water (2x 100 g, tempered to 25 °C) and (via the reactor) twice with a mixture of 1-propanol (2x 67 g, cooled to 0 °C) and water (2x 33 g cooled to 0 °C). Naloxone was obtained as a grayish to brownish solid and dried in a drying oven at 55 °C and pressure of p ≤ 5000 Pa for 16 hours. After drying was completed, the yield of naloxone was 87 % (relative to the amount of noroxymorphone used as starting material) and its purity was 98.9 area-% (measured by HPLC).

It was therefore found by comparing the results of Example 3 above and of present Example 4, that the naloxone obtained by the method of making according to the present invention (i.e. in Example 3 above) is received in higher purity. It is highlighted that 3O-allylnaloxone of formula III was contained in the isolated naloxone in a quantity of 0.8 area-% resulting from Example 4 and in a quantity of only 0.1 area-% resulting from Example 3 (measured by HPLC in each case).

### Example 5: Method of making naloxone hydrochloride according to the present invention

2 g of naloxone (for preparation see Example 3) and optionally butylated hydroxytoluene are mixed with 1-propanol and water. The mixture is heated to 75 °C and stirred until a clear solution is formed. Concentrated hydrochloric acid (38 %, approx. 0.7 g) is then added to the solution. After crystallization has commenced, the resulting suspension is cooled to about 0 °C. The crude title compound is isolated (as dihydrate) and washed with a mixture of 1-propanol and water and optionally dried.

## Claims

1. Crystalline naloxone-dimethylformamide solvate.

2. Crystalline naloxone-dimethylformamide solvate according to claim 1, which
- in an X-ray powder diffraction diagram at 25 °C and using Cu Ka-radiation, displays at least three of the following reflections, quoted as 20-values in °: 6.55 ± 0.2, 10.90 ±0.2, 13.00 ±0.2, 14.06 ±0.2, 15.7 ±0.2, 19.10 ±0.2, 21.09 ± 0.2, 21.77 ± 0.2, 22.79 ± 0.2, and 26.11 ± 0.2;
preferably wherein the crystalline naloxone-dimethylformamide solvate displays all of the following reflections, quoted as 20-values in °: 6.55 ± 0.2, 10.90 ± 0.2, 13.00 ± 0.2, 14.06 ± 0.2, 15.7 ± 0.2, 19.10 ± 0.2, 21.09 ± 0.2, 21.77 ± 0.2, 22.79 ± 0.2, and 26.11 ± 0.2.

3. Crystalline naloxone-dimethylformamide solvate according to anyone of claims 1 or 2, characterized or further **characterized by**:
- an X-ray powder diffraction diagram which is essentially similar to the X-ray powder diffraction diagram shown in Fig. 1,
preferably wherein the crystalline naloxone-dimethylformamide solvate is **characterized by** an X-ray powder diffraction diagram as shown in Fig. 1;
and/or
- a differential scanning calorimetry diagram as shown in Fig. 2;
and/or
- a melting point in the range of from ≥ 84 to ≤ 95 °C, preferably of from ≥ 87 to ≤ 92 °C, determined by differential scanning calorimetry at a heating rate of 4 °C/min;
and/or
- an ¹H-NMR diagram as shown in Figs. 3a, 3b and 3c, all referring to an ¹H-NMR spectrum measured in DMSO-d₆ at 23 °C;
and/or
- an ¹H-NMR spectrum measured in DMSO-d₆ at 23 °C, which shows at least the following signals in ppm: 9.2; 8.0; 6.5; 5.9; 5.24; 5.15; 5.0; 4.8; 3.1; 3.0; 3.0-2.8; 2.7; 2.5-2.4; 2.3; 2.1; 2.0; 1.7; 1.5; 1.3.

4. Crystalline naloxone-dimethylformamide solvate according to anyone of the preceding claims, comprising
- a total amount of naloxone in the range of from ≥ 50 to ≤ 90 wt.-%, preferably of from ≥ 75 to ≤ 85 wt.-%, based on the total weight of the crystalline naloxone-dimethylformamide solvate,
and/or
- a total amount of dimethylformamide in the range of from ≥ 10 to ≤ 50 wt.-%, preferably of from ≥ 15 to ≤ 25 wt.-%, based on the total weight of the crystalline naloxone-dimethylformamide solvate.

5. Method of making naloxone of formula I 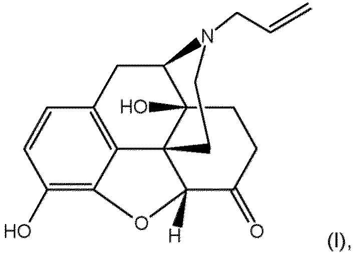 comprising the steps
| | |
|---|---|
| S1) | providing or preparing noroxymorphone of formula II |

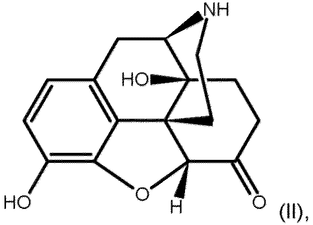
or a salt or a solvate thereof,
S2) reacting the noroxymorphone of formula II, or the salt or solvate thereof, in a liquid reaction medium comprising dimethylformamide, in the presence of a base, with an allyl halide, to receive a first reaction mixture;
S3) to receive a second amount of crystalline naloxone-dimethylformamide solvate as a precipitated solid,
i) seeding the first reaction mixture received in step S2) with a first amount of a crystalline naloxone-dimethylformamide solvate according to any of claims 1 to 4 or 11,
or
ii) stirring the first reaction mixture received in step S2) for a time period sufficient for a second amount of crystalline naloxone-dimethylformamide solvate to precipitate as a solid,
and/or
adjusting the temperature of the first reaction mixture received in step S2) to a temperature in the range of from -60 °C to 30 °C,
and
S4) adding to the crystalline naloxone-dimethylformamide solvate received in step S3) as precipitated solid a solvent or solvent mixture suitable to release naloxone of formula I from said crystalline naloxone-dimethylformamide solvate.

6. Method according to claim 5, wherein
- step S3) comprises variant i) and preferably further comprises adjusting the temperature of the seeded first reaction mixture received in step S2) to a temperature in the range of from -60 °C to 120 °C, preferably of from -20 °C to 40 °C, more preferably cooling the seeded first reaction mixture to a temperature in the range of from -15 °C to 20 °C, even more preferably of from -15 °C to 15 °C and yet even more preferably of from -15 °C to 5 °C;
and/or
- the allyl halide in step S2) is selected from the group consisting of allyl chloride, allyl bromide, allyl iodide and mixtures thereof, preferably wherein the allyl halide in step S2) comprises or is allyl bromide;
and/or
- the first reaction mixture in step S2) and/or the seeded first reaction mixture in step S3) comprises 3O-allylnaloxone of formula III 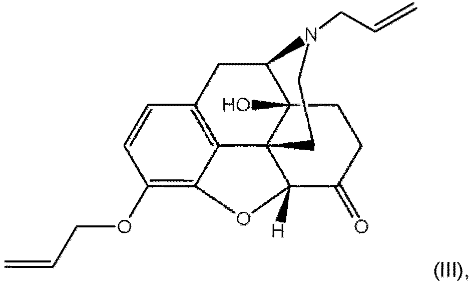 preferably as an impurity.

7. Method according to any of claims 5 or 6, wherein
- the solvent or solvent mixture suitable to release naloxone of formula I from the precipitated solid crystalline naloxone-dimethylformamide solvate in step S4) is selected from the group consisting of
- water,
- an alkyl alcohol having 1 to 4 carbon atoms, preferably selected from the group consisting of methanol, ethanol, 1-propanol, isopropanol, n-butanol, 2-methyl-1-propanol and mixtures thereof, more preferably the alkyl alcohol having 1 to 4 carbon atoms comprises or is 1-propanol;
and
- mixtures thereof
and/or
- step S4) comprises adjusting the pH of the mixture, received after addition of the solvent or solvent mixture suitable to release naloxone of formula I from said crystalline naloxone-dimethylformamide solvate, to a value in the range of from 8.5 to 10, preferably of from 9.0 to 9.5, preferably measured at 0 °C;
and/or
- the method further comprises the step
S5) separating solid naloxone of formula I as received from step S4) and optionally drying it.

8. Crystalline naloxone, which
- in an X-ray powder diffraction diagram at 25 °C and using Cu Ka-radiation, displays at least three of the following reflections, quoted as 20-values in °: 10.46 ± 0.2, 11.23 ± 0.2, 11.68 ± 0.2, 13.61 ± 0.2, 14.53 ± 0.2, 16.27 ± 0.2, 16.63 ± 0.2, 17.45 ± 0.2, 18.05 ± 0.2, 20.31 ± 0.2, 20.95 ± 0.2;
preferably wherein the crystalline naloxone-dimethylformamide solvate displays all of the following reflections, quoted as 20-values in °: 10.46 ± 0.2, 11.23 ± 0.2, 11.68 ± 0.2, 13.61 ± 0.2, 14.53 ± 0.2, 16.27 ± 0.2, 16.63 ± 0.2, 17.45 ± 0.2, 18.05 ± 0.2, 20.31 ± 0.2, 20.95 ± 0.2,
and/or
- is obtainable or obtained by a method of making naloxone according to any of claims 5 to 7.

9. Method of making a naloxone salt, preferably naloxone hydrochloride, comprising the steps
| | |
|---|---|
| S6) | providing crystalline naloxone according to claim 8 or preparing naloxone of formula I by the method according to any of claims 5 to 7, and |
| S7) | reacting the naloxone of formula I as received from step S6) with an acid, preferably with hydrochloric acid. |

10. Method of making a first amount of a crystalline naloxone-dimethylformamide solvate, preferably according to any of claims 1 to 4, comprising the steps
| | |
|---|---|
| S11) | providing or preparing naloxone, or a salt or a solvate thereof, wherein any provided or prepared solvate of naloxone is different from crystalline naloxone-dimethylformamide solvate, |
| S12) | dissolving or suspending the naloxone, or the salt or solvate thereof, provided or prepared in step S11) in a liquid reaction medium comprising dimethylformamide, for a time sufficient so that the crystalline naloxone-dimethylformamide is formed and |
| S13) | preferably isolating the crystalline naloxone-dimethylformamide which has formed in step S12) and optionally drying it. |

11. Crystalline naloxone-dimethylformamide solvate, obtainable or obtained by a method according to claim 10.

12. Method of making a second or further amount of a crystalline naloxone-dimethylformamide solvate, preferably according to any of claims 1 to 4 or 11, comprising the steps
| | |
|---|---|
| S21) | providing or preparing, in a liquid reaction medium comprising dimethylformamide, naloxone of formula I, as defined in claim 5, or a salt or a solvate thereof, wherein any provided or prepared solvate of naloxone is different from naloxone-dimethylformamide solvate, |
| | to receive a second reaction mixture; |
| and | |
| S22) | seeding the second reaction mixture received in step S21) with a first amount of crystalline naloxone-dimethylformamide solvate according to any of claims 1 to 4 or 11, and preferably cooling the seeded second reaction mixture, to receive a second amount of crystalline naloxone-dimethylformamide solvate as a precipitated solid. |

13. Method according to claim 12, wherein the second reaction mixture in step S21) and/or the seeded second reaction mixture in step S22) comprises 3O-allylnaloxone of formula III as defined in claim 6, preferably as an impurity.

14. Use of a crystalline naloxone-dimethylformamide solvate, preferably according to any of claims 1 to 4 or 11, as a reagent for crystallizing, recrystallizing and/or purifying naloxone or a salt or a solvate thereof.

15. Use of dimethylformamide for preparing a crystalline naloxone-dimethylformamide solvate from naloxone.

16. Mixture, preferably suspension, comprising dimethylformamide and a crystalline naloxone-dimethylformamide solvate, preferably according to any of claims 1 to 4 or 11.
